# EUROPÄISCHE PATENTSCHRIFT

(11) **EP 1 120 130 B1**
(45) Veröffentlichungstag und Bekanntmachung des Hinweises auf die Patenterteilung: **14.12.2005**
(21) Anmeldenummer: 01100932.1
(22) Anmeldetag: 17.01.2001
(51) Int. Cl.: A61N 1/37, A61N 1/372, G11C 13/06, H01L 27/144

(54) **Medizinisches Geräteimplantat**
Medical implantable device
Dispositif médical implantable

(30) Priorität: 25.01.2000 DE 10002932
(43) Veröffentlichungstag der Anmeldung: 01.08.2001
(73) Patentinhaber: BIOTRONIK Mess- und Therapiegeräte GmbH & Co Ingenieurbüro Berlin, 12359 Berlin (DE)
(72) Erfinder: Busch, Ulrich, 10318 Berlin (DE); Niewalda, Gregor, 91054 Erlangen (DE)
(74) Vertreter: Hübner, Gerd

(56) Entgegenhaltungen:
- US-A- 5 701 894
- US-A- 5 883 397
- US-A- 5 941 906
- US-A- 5 954 666

## Beschreibung

Die Erfindung betrifft ein medizinisches Geräteimplantat, insbesondere ein kardiologisches Implantat wie einen Herzschrittmacher oder Defibrillator, mit den im Oberbegriff des Anspruches 1 angegebenen Merkmalen.

Derartige Geräteimplantate weisen im allgemeinen eine zentrale Steuereinheit zur Steuerung der geräteinternen und externen Funktionen auf. Konkret für einen Herzschrittmacher bedeutet dies, daß beispielsweise ein Mikroprozessor die Schrittmacher-internen Steuerungsabläufe, die Verarbeitung der von Meßelektroden im Herzen gewonnenen EKG-Daten und die Ansteuerung von Treibereinheiten für die Abgabe von elektrischen Stimulationsimpulsen an das Herz übernimmt.

Zur Wahrnehmung dieser Funktionen benötigt die zentrale Steuereinheit unter anderem eine Speichereinheit, umfassend Programm-, Arbeits- und/oder Datenspeicher. Die Auslegung und Notwendigkeit der verschiedenen Speichertypen richtet sich nach den Funktionen des Implantates.

Schließlich ist naturgemäß aufgrund ihres Einsatzortes für solche Geräteimplantate eine netzunabhängige Spannungsversorgung z.B. in Form einer Batterie vorzusehen.

Ein Kernproblem bei bis heute üblichen Geräteimplantaten ist die Tatsache, daß als Speichereinheiten Halbleiter-Speicher-Bausteine eingesetzt werden. Diese stellen zwar an sich eine ausreichende Speicherkapazität zur Verfügung, um auch komplexe Funktionen der Geräteimplantate zu unterstützen. Allerdings weisen solche Speicherbausteine auch einen erheblichen Stromverbrauch auf, der sich in der Praxis bei langfristig zu implantierenden Geräten begrenzend auf die Größe der verwendbaren Speicherbausteine auswirkt. Dies wiederum limitiert die Funktionalität des Gerätes, was aufgrund des immer komplexer werdenden Funktionsspektrums solcher Implantate der Entwicklungstendenz natürlich zuwider läuft. Ferner wird mit den zunehmend an Bedeutung gewinnenden Halbleiter-Speichern auf der Basis von Sub-Mikron-Technologien zwar einerseits deren Flächenbedarf immer geringer, andererseits wächst deren statischer Stromverbrauch im Verhältnis dazu überproportional an.

An sich sind im Stand der Technik zwar Speichermedien bekannt, die auf der Basis von magnetischen Effekten nahezu ohne Stromverbrauch arbeiten und daher als nicht-flüchtige Speicher gelten können. Diese haben sich jedoch für die einschlägigen Geräteimplantate aufgrund ihrer aus anderen Gründen begrenzten Speicherkapazität als wenig tauglich erwiesen.

Zur Lösung der vorstehenden Problematik schlägt die Erfindung nun vor, für die Speichereinheit auf einem optischen Speichermedium beruhende, nicht-flüchtige Schreib-/Lese-Speicher einzusetzen, die die gespeicherten Informationen energielos erhalten.

Diese Art von Speicher weist erhebliche Vorteile insbesondere im Hinblick auf den Einsatz in netzunabhängig zu betreibenden Geräteimplantaten auf. So wird Energie lediglich zum Auslesen oder Ändern des Speicherinhalts verbraucht, nicht jedoch für die Informationshaltung. Dadurch wird der Energieverbrauch eines solchen Implantates drastisch eingeschränkt, bei vorgegebener Energiekapazität kann also umgekehrt die Speicherkapazität erhöht werden. Ferner können optische Speicher in sehr unterschiedlichen Konfigurationen realisiert werden, die speziell auf ihren Einsatz in medizinischen Geräteimplantaten zugeschnitten sein können. Bei der Silizium-Speichertechnologie ist diese Variabilität nicht in diesem Maße gegeben.

Die Ansprüche 2 und 3 kennzeichnen eine erste Variante für das optische Speichermedium, das ein Polymer mit optisch anregbaren Molekülstrukturen sein kann. Die Funktion einer Speicherzelle beruht also beispielsweise auf der Ausrichtung eines Moleküls im Polymermaterial, die durch Lichteinstrahlung veränderbar ist. Die jeweilige Ausrichtung kann sich in optischen Eigenschaften, wie beispielsweise der Polarität oder dem Absorptionskoeffizienten des Materials niederschlagen. Über diese physikalischen Eigenschaften kann die Information von außen abgetastet werden. Speichern und Auslesen von Information kann insbesondere durch Einstrahlung unterschiedlicher optischer Impulse erfolgen. So kann mit untergrenzwertigen Impulsen beispielsweise der Absorptionskoeffizient einer optischen Speicherzelle und damit ihr Informationsinhalt abgetastet werden. Wird ein optischer Impuls über den definierten Grenzwert eingestrahlt, so ist die Molekülstruktur anregbar und kann durch Änderung der Ausrichtung einen anderen Informationsinhalt, z.B. in Form eines geänderten Absorptionskoeffizienten, annehmen.

Die Ansteuerschaltung eines solchen polymerischen Schreib-/LeseSpeichers kann vorteilhafterweise mit polymerischen Transistoren realisiert werden, so daß sich die Schnittstelle zwischen den Silizium-basierten Funktionseinheiten des Implantates und der optischen Speichereinheit auf wenige Kontakte reduzieren kann.

Als zweite Alternative für ein optisches Speichermedium gibt Anspruch 5 ein Halbleitermaterial mit optisch anregbaren Energiesenken in einem Leitungsband an. Durch dieses Konzept lassen sich Elektronen zunächst durch optische Anregungen in das Leitungsband anheben, wo sie durch die Energiesenken energielos festgehalten werden. Damit kann also ebenfalls eine Speicherzelle realisiert werden, wobei das Abtasten der Information und das Anheben der Elektronen in das Leitungsband und umgekehrt dessen Rücktransformation in den Ausgangszustand wiederum über die Einstrahlung von Licht einer bestimmten Wellenlänge erfolgt.

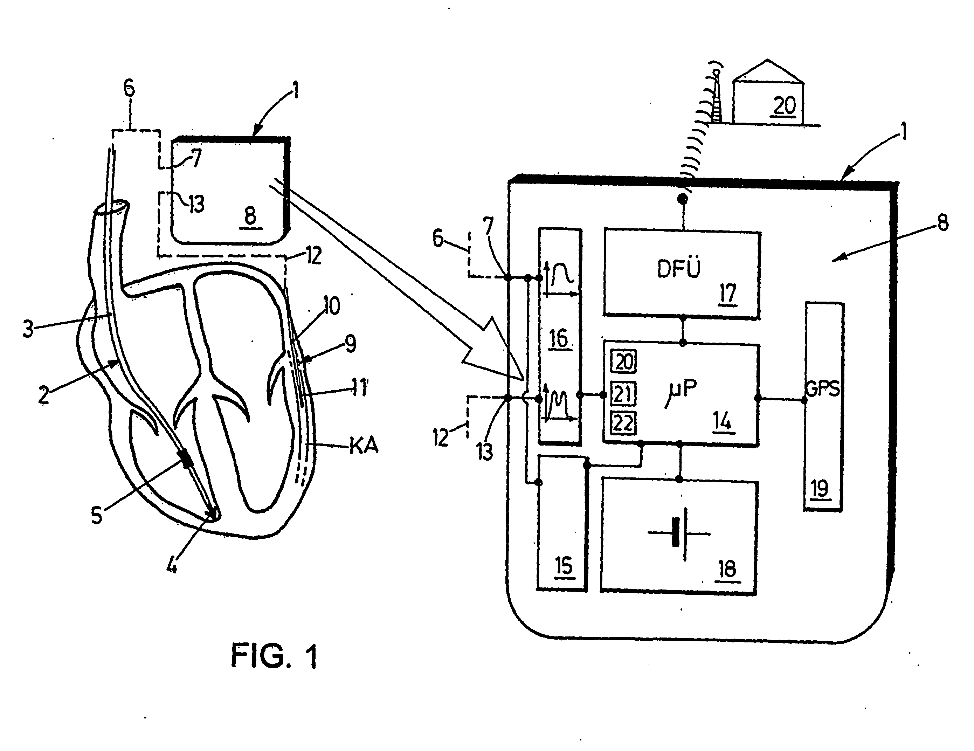

Weitere Merkmale, Einzelheiten und Vorteile der Erfindung ergeben sich aus der nachfolgenden Beschreibung, in der ein Ausführungsbeispiel anhand der beigefügten Zeichnung näher erläutert wird. Diese
- Fig. 1: zeigt eine schematische, teilweise als Blockdiagramm angelegte Darstellung einer implantierbaren Detektionsvorrichtung zur Erkennung von Ischämie-Zuständen des Herzens.

In Fig. 1 ist eine implantierbare Detektionsvorrichtung schematisch dargestellt, die ein nach Art eines Herzschrittmachers ausgestaltetes Implantatgehäuse 1 aufweist. Dieses ist in den Brustkorb eines Ischämie-bedrohten Patienten eingepflanzt. Die Detektionsvorrichtung ist ferner mit einer Elektroden-Sensoranordnung 2 versehen, die aus einem Katheter 3 mit einer Stimulationselektrode 4 zur Abgabe eines Stimulationsimpulses an das Herz und einer unipolaren, fraktal beschichteten Schrittmacherelektrode als Sensorelektrode 5 zur Detektion z.B. der ventrikulär evozierten Reizantwort des Herzens besteht. Die Elektroden-Sensoranordnung 2 ist durch eine strichliert angedeute Koppelleitung 6 über einen entsprechenden Eingang 7 am Implantatgehäuse 1 mit der im vergrößerten Ausschnitt der Fig. 1 dargestellten Steuereinrichtung 8 verbunden.

Es ist ferner eine Blutströmungs-Sensoranordnung 9 vorgesehen, die im gezeigten Ausführungsbeispiel aus einem in eine Koronararterie KA eingeführten Katheter 10 mit einem Strömungsmeßsensor 11 beispielsweise auf der Basis eines Ultraschall-Doppler-Meßkopfes oder einer Impedanz-Meßelektrode besteht. Auch die Blutströmungs-Sensoranordnung 9 ist über eine Koppelleitung 12 mit einem entsprechenden Eingang 13 der Steuereinrichtung 8 verbunden.

Die Steuereinrichtung 8 weist als zentrales Steuerteil einen Mikroprozessor 14 auf, der alle weiteren Bauteile der Steuereinrichtung 8 über ein entsprechendes Betriebsprogramm in üblicher Weise steuert. Zur Speicherung des Betriebsprogrammes ist dabei ein Programmspeicher 20 dem Mikroprozessor 14 zugeordnet. Ein Arbeitsspeicher 21 dient bei der Durchführung aller geräteinternen Steuerungsfunktionen und der Ablaufsteuerung für die externen Funktionalitäten der Zwischenspeicherung der entsprechenden Arbeitsdaten. Ferner ist ein Datenspeicher 22 vorgesehen, der beispielsweise bestimmte Programmiereinstellungen der Detektionsvorrichtung gespeichert hält.

Die Speicher 20, 21, und 22 sind als nicht-flüchtige Schreib-/Lese-Speicher ausgeführt, die auf einem optischen Speichermedium beruhen und die gespeicherten Informationen energielos erhalten. Die Speicher sind als sogenannte "organische Speicher" auf der Basis eines Polymerschichtmaterials mit optisch anregbaren Molekülstrukturen ausgebildet. Der grundsätzliche Aufbau solcher Speicher und ihre Wirkungsweise wurden in der Beschreibungseinleitung bereits dargelegt, worauf zur Vermeidung von Wiederholungen verwiesen wird. Nähere Angaben über den technischen Stand dieser Speicher sind ferner einem Artikel aus der Computer-Fachzeitschrift c't, Nr. 3/1998, S. 18, Ulrike Kuhlmann, Dr. Jürgen Rink "Terabytes in Plastikfolie - organische Massenspeicher vor der Serienproduktion?" entnehmbar.

Zur Energieversorgung der geräteinternen Einheiten 14, 15, 16, 17 und 19, wie sie bereits erörtert bzw. im folgenden noch näher beschrieben werden, ist eine Batterie 18 vorgesehen.

Über die Steuereinrichtung 8 wird nun eine Stimulationseinrichtung 15 tiviert, so daß diese über die Stimulationselektrode einen Impuls zur Erzeugung beispielsweise einer ventrikulär evozierten Reizantwort (VER) aktiviert.

Zur Auswertung und Weitergabe der von den Sensoranordnungen 2, 9 gelieferten Signale über die ventrikulär evozierte Reizantwort und die in der Koronararterie KA gemessene Blutströmung ist die Erfassungseinrichtung 16 vorgesehen. So wird beispielsweise bei einer Absenkung der Amplitude der VER aufgrund einer Ischämie bei Unterschreiten einer bestimmten ersten Schwelle - also wenn noch kein akuter Ischämie-Anfall vorliegt - an einen Herzschrittmacher, als dessen Bestandteil die erfindungsgemäße implantierbare Detektionsvorrichtung ausgelegt sein kann, der Befehl gegeben, die Stimulationsrate nach oben hin zu begrenzen. Dadurch wird der Stoffwechselbedarf des Herzmuskelgewebes klein gehalten, wodurch einer weiteren Verschärfung des ischämischen Zustandes entgegengewirkt werden kann. Bei einem derartigen Ischämie-Zustand stellt die Blutströmungs-Sensoranordnung 9 noch keine signifikante Erniedrigung der Versorgung des Myocards fest.

Bei einer weiteren Absenkung der Amplitude der VER unter eine zweite Schwelle und ggf. bei Messung eines Absinkens der Blutströmung in der Koronararterie KA mit Hilfe der Sensoranordnung 9 können nun Notfallmaßnahmen verschiedenster Grade auszulösen sein. Hierzu ist die Detektionsvorrichtung mit einer Datenfernübertragungseinrichtung 17 auf der Basis in der Herzschrittmachertechnik üblicher Telemetrie-Techniken versehen, mittels der in der Steuereinrichtung 8 erfaßter und über die Erfassungseinrichtung 16 ausgewerteter und weiterverarbeiteter Signaldaten zu einer nicht näher dargestellten Außen- oder Basisstation 20, z. B. einem den Patienten betreuenden Herzzentrum weitergeleitet werden können. Von dort aus können ebenfalls über die Datenfernübertragungseinrichtung 17 beispielsweise weitere Messungen mit Hilfe der Detektionsvorrichtung oder des damit gekoppelten Herzschrittmachers initiiert werde. Der Patient kann ferner von der Basisstation 20 zum Arzt einbestellt werden.

Ferner ist in der Detektionsvorrichtung eine mit der Datenfernübertragungseinrichtung 17 gekoppelte Navigations-Einrichtung 19 auf der Basis des üblichen GPS- oder eines GSM-Systems integriert. Damit kann insbesondere bei einem akuten Ischämie-Anfall die Position des Patienten bestimmt und der Basisstation 20 mitgeteilt werden. Diese kann dann sofort einen Notfalldienst verständigen, der die geografische Position des Patienten über die Satelliten-Navigations-Einrichtung kennt. Extrem schnelle und selektiv an die Schwere des Ischämie-Anfalles angepaßte Notfallmaßnahmen sind damit gewährleistet.

## Patentansprüche

1. Medizinisches Geräteimplantat, insbesondere kardiologisches Implantat, wie Herzschrittmacher oder Defibrillator, mit
- einer zentralen Steuereinheit (14) zur Steuerung der geräteinternen und externen Funktionen,
- einer Speichereinheit, umfassend Programm-, Arbeits- und/oder Datenspeicher (20, 21, 22) für die zentrale Steuereinheit (14), und
- einer netzunabhängigen Spannungsversorgung (18) für das Geräteimplantat,
**dadurch gekennzeichnet, daß** die Speichereinheit (20, 21, 22) versehen ist mit
- mindestens einem auf einem optischen Speichermedium beruhenden, nicht-flüchtigen Schreib-/Lese-Speicher, der die gespeicherten Informationen energielos erhält.

2. Geräteimplantat nach Anspruch 1, **dadurch gekennzeichnet, daß** das optische Speichermedium ein Polymer mit optisch anregbaren Molekülstrukturen ist.

3. Geräteimplantat nach Anspruch 2, **dadurch gekennzeichnet, daß** die Molekülstrukturen des optischen Speichermediums selektiv durch Einstrahlung unterschiedlicher optischer Impulse hinsichtlich der von ihnen gespeicherten Informationen auslesbar oder zur Neuspeicherung von Information schaltbar sind.

4. Geräteimplantat nach einem der Ansprüche 1 bis 3, **dadurch gekennzeichnet, daß** die Ansteuerschaltung des Schreib-/Lesespeichers auf der Basis polymerischer Transistoren realisiert ist.

5. Geräteimplantat nach Anspruch 1, **dadurch gekennzeichnet, daß** das optische Speichermedium ein Halbleitermaterial mit optisch anregbaren Energiesenken in einem Leitungsband ist.

## Claims

1. An implantable medical device, more particularly an implantable cardiac device, such as a cardiac pacemaker or defibrillator, having
- a central control unit (14) for controlling the internal and external functions of the device,
- a memory unit comprising program memory, main and/or data memory (20, 21, 22) for the central control unit (14), and
- an off-the-line power supply (18) for the implantable device,
**characterized in that** the memory unit (20, 21, 22) incorporates
- at least one non-volatile read-write memory that is based on an optical storage medium and holds the stored information energy-free.

2. An implantable device according to claim 1, **characterized in that** the optical storage medium is a polymer with optically excitable molecule structures.

3. An implantable device according to claim 2, **characterized in that** the molecule structures of the optical storage medium are selectively readable or switchable for writing new information through the emittance of various optical pulses onto the memory medium.

4. An implantable device according to claims 1 through 3, **characterized in that** the control circuit of the read-write memory is implemented on the basis of polymeric transistors.

5. An implantable device according to claim 1, **characterized in that** the optical storage medium is a semiconductor material with optically excitable energy sinks in a conductance band.

## Revendications

1. Appareil médical implantable, notamment implant cardiologique tel qu'un stimulateur cardiaque ou un défibrillateur, comportant
- une unité de commande centrale (14) pour la commande des fonctions internes et externes de l'appareil,
- une unité de mémorisation, comportant une mémoire de programme, de travail et/ou de données (20, 21, 22) pour l'unité de commande centrale (14), et
- une alimentation en tension (18) indépendante du réseau pour l'appareil implantable,
**caractérisé en ce que** l'unité de mémorisation (20, 21, 22) est dotée
- d'au moins une mémoire d'enregistrement / de lecture non volatile a base d'un support de mémorisation optique, qui reçoit les données mémorisées sans énergie.

2. Appareil implantable selon la revendication 1, **caractérisé en ce que** le support de mémorisation optique est un polymère avec des structures moléculaires pouvant être excitées par voie optique.

3. Appareil implantable selon la revendication 2, **caractérisé en ce que**, moyennant l'envoi de différentes impulsions optiques, les structures moléculaires du support de mémorisation optique peuvent être lues sélectivement en ce qui concerne les informations mémorisées par elles ou commutées pour une nouvelle mémorisation d'informations.

4. Appareil implantable selon l'une des revendications 1 à 3, **caractérisé en ce que** le circuit de commande de la mémoire d'enregistrement / de lecture est réalisé à base de transistors polymères.

5. Appareil implantable selon la revendication 1, **caractérisé en ce que** le support de mémorisation optique est un matériau semi-conducteur avec des dépressions énergétiques pouvant être excitées par voie optique dans une bande de conduction.
